# EUROPEAN PATENT APPLICATION

(11) **EP 1 974 728 A1**
(43) Date of publication of application: **01.10.2008**
(21) Application number: 06730812.2
(22) Date of filing: 31.03.2006
(51) Int. Cl.: A61K 31/23, A61K 31/231, A61K 31/232, A61P 3/06, A61P 9/10, A61P 3/04, A61P 3/10, A61P 9/12, A23L 1/30, A23K 1/16

(54) **LIPID METABOLISM IMPROVING COMPOSITION**

(30) Priority: 30.09.2005 JP 2005288401
(71) Applicant: The Nisshin OilliO Group, Ltd., Tokyo 104-8285 (JP)
(72) Inventor: YANAGITA, Teruyoshi, Saga8408502 (JP); NAGAO, Koji, Saga 8408502 (JP); WANG, Yu-Ming, Saga 8408502 (JP); INOUE, Nao, Saga 8408502 (JP); ARAO, Keisuke, Saga 8408502 (JP); IWATA, Toshio, Tokyo 1048285 (JP)
(74) Representative: Müller-Boré & Partner Patentanwälte
(86) International application number: PCT/JP2006/306864
(87) International publication number: WO 2007/039945

(57) **Abstract**

Disclosed is a composition for improving lipid metabolism. The composition for improving lipid metabolism comprises a fatty acid menthol ester as an active ingredient.

## Description

### [CROSS-REFERENCE TO RELATED APPLICATIONS]

The present application is a patent application claiming priority based on Japanese Patent Application No. 288401/2005 under the Paris Convention, and, thus, the disclosure thereof is totally incorporated herein.

### [TECHNICAL FIELD]

The present invention provides a composition for lipid metabolism improvement, food and drink, a health (supplementary) food, a feed, or a pharmaceutical composition, comprising a fatty acid menthol ester.

### [BACKGROUND ART]

In omnivorous mammals, lipids obtained from food and drink are absorbed through small intestine, are passed through a lymphoid lineage, and are utilized as chylomicrons in body tissues located outside the liver. Lipids from the liver are brought to a part of ultra-low-density lipoproteins (hereinafter abbreviated to "VLDL"), are passed through blood, are transported to body tissues, undergo the action of lipoprotein lipase on the body tissue surface to be degraded to free fatty acids which are then consumed as energy. In adipose tissues (adipose cells), the hydrolysis and (re)esterization of triacylglycerol irreversibly proceed, whereby accumulation and decomposition of lipids take place.

Apolipoproteins may be mentioned as an important factor in lipid metabolism. Apolipoproteins are special plasma proteins constituting lipoproteins and function to solubilize lipids in blood and to carry the solibilized lipids into the body tissue. Further, the apolipoproteins function, for example, to activate and inhibit enzymes involved in lipid metabolism and further function as a ligand for a cross-reaction with a lipoprotein receptor in the tissue and thus have been regarded as a plsama component important in the lipid metabolism.

Apolipoprotein B (particularly B-100) as one of major components of apolipoproteins is synthesized in the liver and functions to associate, for example, with apolipoprotein E, apolipoprotein C, and triacylglycerol (hereinafter abbreviated to "TG"), phospholipids, cholesterol (hereinafter abbreviated to "C"), and cholesterol esters (hereinafter abbreviated to "CE") to form VLDL and to carry TG and C synthesized in the liver and CE to peripheral tissues. In particular, apolipoprotein B-100 is regarded as acting as a ligand for LDL receptors. It is said that enhanced secretion of apolipoprotein B causes an increase in the level of VLDL in blood, an increase in the level of low-density lipoprotein (hereinafter abbreviated to "LDL"), an increase in TG level of blood, and an increase in levels of C and CE in blood. Further, oil droplets are said to be accumulated in small intestine and liver. Consequently, the enhanced secretion of apolipoprotein B is causative of various diseases such as the so-called hypertriacylglycerolemia and hypercholesterolemia. Accordingly, when the apolipoprotein B (particularly B-100) can be inhibited, it is expected that the level of TG, C and CE in blood are lowered and, at the same time, the level of LDL in blood is lowered contributing to the prevention, amelioration or treatment of lifestyle-related illness typified, for example, by atherosclerosis, obesity, diabetes, and hypertension, coronary artery diseases and cerebral artery diseases.

On the other hand, apolipoprotein A is synthesized in the liver and intestinal tract and associates, for example, with apolipoprotein E and apolipoprotein C to form high-density lipoproteins (hereinafter abbreviated to as "HDL") and to reversely transfer excess TG, C and CE from peripheral tissues or macrophages to the liver. The enhanced secretion of apolipoprotein A (particularly A-1) activates lecithin:cholesterol acyltransferase (hereinafter abbreviated to "LCAT"), which are enzymes capable of converting C released on HDL to CE, and play an important role relative to a reverse transfer system of C. On the other hand, a lowering in apolipoprotein A is said to increase the risk of ischemic heart diseases. Further, it is considered that the proportion of LDL to HDL increases correlatively with increasing the proportion of apolipoprotein B (particularly B-100) to apolipoprotein A. This results in the development of symptoms of diseases such as lifestyle-related illness, coronary artery diseases, and cerebral artery diseases.

Several apolipoprotein B secretion inhibitors have recently been exemplified, and pharmaceutical compositions or foods and drinks using them have been proposed. For example, α-linolenic acid and 10trans,12-conjugated linolenic acid (Yanagida et al., Food Res. Intern., 31, 403, 1999), taurine and γ-oryzanol (Yanagida et al., Proceedings of Anuual Meeting of Japan Society for Bioscience, Biotechnology, and Agrochemistry 1998, 3E2a10), onion-derived sulfoamino sulfoxide (Yanagida et al., Proceedings of Anuual Meeting of Japan Society for Bioscience, Biotechnology, and Agrochemistry 2000, 2G020α), NK-104 "3-Hydroxy-3-Methlyglutaryl CoenzymeA Reductase Inhibitor" (Yanagida et al., Current Therapeutic Research, 60, 423, 1999), anhydrous hydrogen chloride of 4'-trifluoromethylbiphenyl-2-carboxylic acid-[2-(2-acetylaminoethyl-1,2,3,4-tetrahydroisoquinolin-6-yl) amide (Japanese Patent Laid-Open No. 60557/1999), and 4'-trifluoromethyl-biphenyl-2-carboxylic acid-[2-(1H-[1,2,4-triazol-3-ylmethyl)]-(1,2,3,4-tetrahydro-isoq uinolin-6-yl)-amide (Japanese Translation of PCT Publication No. 510483/2000) have been proposed as the apolipoprotein B secretion inhibitor.

According to studies conducted by the present inventors, at the present time, any substance useful as the apolipoprotein B secretion inhibitor has not hitherto been found. Further, any substance, which inhibits the secretion of apolipoprotein B and, at the same time, accelerates the secretion of apolipoprotein A, has not hitherto been proposed.

### [SUMMARY OF THE INVENTION]

At the time of the present invention, the present inventors have found that fatty acid menthol esters can inhibit apolipoprotein B secretion and that fatty acid menthol esters can inhibit apolipoprotein B secretion and, at the same time, can accelerate the section of apolipoprotein A. At the time of the present invention, based on the above finding, the present inventors have found that a composition for improving lipid metabolism, food and drink, a health (supplementary) food, a feed, and a pharmaceutical composition, comprising an effective amount of a fatty acid menthol ester can be provided. The present invention has been made based on such finding.
Thus, according to the present invention, there is provided a composition for improving lipid metabolism, comprising an effective amount of a fatty acid menthol ester.

### [BRIEF DESCRIPTION OF THE DRAWINGS]

[Fig. 1] Fig. 1 is a diagram showing the amount of apolipoprotein B (B100) secreted from human liver-derived HepG2 cells.
[Fig. 2] Fig. 2 is a diagram showing the amount of apolipoprotein A (A1) secreted from human liver-derived HepG2 cells.

### [BEST MODE FOR CARRYING OUT THE INVENTION]

### Composition for improving lipid metabolism/lipid metabolism improving agent

### Definition

The term "lipid metabolism improvement" as used herein refers to, in lipid metabolism, for example, degradation (promotion of degradation) of lipid and its components (fatty acids) present in body tissues or blood and lymph (lymphoid tissues), prevention or suppression of accumulation of lipid and its components in body tissues (particularly fatty tissues), and a reduction in lipid and its components accumulated in body tissues (particularly fatty tissues). Specifically, the term "lipid metabolism improvement" includes prevention or suppression of the presence, in high concentration, of lipid and its components ingested by eating in blood and lymph (lymphoid tissues), a reduction of lipid present in high concentration in blood and lymph (lymphoid tissues), prevention and suppression of accumulation of lipid and its components ingested by eating in body tissues, degradation or a reduction of lipid and its components accumulated in body tissues, or degradation of or a reduction in concentration of lipid and its components present in blood upon release, into blood, of lipid and its components accumulated in body tissues. Accordingly, fatty acid menthol esters according to the present invention have the following various functions (applications) for lipid metabolism improvement.

### Fatty acid menthol ester

The fatty acid menthol ester as an active ingredient of the composition for lipid metabolism improvement is represented by general formula (I): 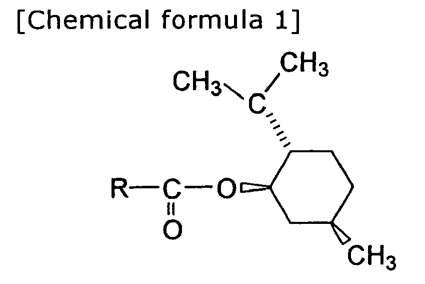 wherein
R represents a saturated fatty acid residue, a straight-chain monoenoic acid residue, a polyenoic acid residue, or a hydroxy acid residue. Here the term "residue" in the straight-chain saturated fatty acid residue, straight-chain monoenoic acid residue, polyenoic acid residue, and hydroxy acid residue refers to such a state that these fatty acids lack for the carboxylic acid group.

In the present invention, the fatty acid constituting the fatty acid menthol ester (hereinafter referred to as "constituent fatty acid") may be of a straight chain or branched chain type regardless of whether the fatty acid is saturated or unsaturated. Specific examples thereof include saturated fatty acids, monoenoic acids, polyenoic acids, or hydroxy acids [CH₃(CH₂)ₙCH(OH)COOH], wherein n is an odd number of 5 or more and 21 or less. The constituent fatty acid is preferably one or at least two fatty acids selected from the group consisting of fatty acids having 8 or more and 24 or less carbon atoms, preferably fatty acids having 16 or more, more preferably 18 or more, and 22 or less carbon atoms.
In a preferred embodiment of the present invention, the constituent fatty acid is one or at least two fatty acids selected from the group consisting of saturated fatty acids such as caprylic acid (octanoic acid), capric acid (decanoic acid), lauric acid (dodecanoic acid), myristic acid (tetradecanoic acid), palmitic acid, stearic acid, arachidic acid, behenic acid, and ligseric acid; monoenoic acids such as palmitoleic acid, oleic acid, elaidic acid, eicosapentaenoic acid, and docosahexaenoic acid; polyenoic acids such as linoleic acid, conjugated linoleic acid, γ-linolenic acid, α-linolenic acid, conjugated linolenic acid, and arachidonic acid; and hydroxy acids such as 2-hydroxylignoceric acid and hydroxynervonic acid. Among them, more preferred constituent fatty acids are fatty acids having 18 or more carbon atoms. The fatty acid menthol ester can be produced by subjecting one or at least two fatty acids and menthol to an esterification reaction.

### Functions and properties

The fatty acid menthol ester can be utilized as a substance for improving lipid metabolism. The functions and properties of the fatty acid menthol ester are specifically as follows. The fatty acid menthol ester inhibits the secretion of apolipoprotein B and/or promotes the secretion of apolipoprotein A. More specifically, the secretion of the apolipoprotein B is inhibited in the liver and/or the secretion of the apolipoprotein A is promoted in the liver and/or intestinal tract.

Accordingly, upon the administration of the fatty acid menthol ester to mammals, the secretion of apolipoprotein B is inhibited, the concentration of triacylglycerol, cholesterol and/or cholesterol ester in blood, lymph (lymphoid tissues) and/or body tissues is lowered, and/or the secretion of apolipoprotein A is promoted, and lecithin:cholesterol acyltransferase ("LCAT") is activated to promote the degradation of cholesterol in blood, lymph (lymphoid tissues) and/or body tissues. That is, the fatty acid menthol ester lowers the concentration of triacylglycerol, cholesterol and/or cholesterol ester in blood, lymph (lymphoid tissues) and/or body tissues, reduces the ingredients accumulated in the body tissues, and further effectively prevent the accumulation of these ingredients in body tissues. Further, the fatty acid menthol ester lowers the concentration of low-density lipoprotein in blood, lymph (lymphoid tissues) and/or body tissues, and/or increases the concentration of high-density lipoprotein in blood, lymph (lymphoid tissues) and/or body tissues.

### Use

The fatty acid menthol ester according to the present invention is utilized as a composition for lipid metabolism improvement and is preferably used for preventing, improving, or treating at least one symptom selected from the group consisting of atherosclerosis, obesity, hypercholesterolemia, hypertriacylglycerolemia, hyperlipidemia, fatty liver, diabetes, and hypertension. The content of the fatty acid menthol ester may be properly determined depending upon applications used. Specifically, the content of the fatty acid menthol ester is approximately more than 0% by weight and not more than 100% by weight based on the total weight of the composition for lipid metabolism improvement. Preferably, the lower limit of the content is about 5% by weight, and the upper limit of the content is about 90% by weight. More preferably, the lower limit of the content is about 50% by weight, and the upper limit of the content is about 80% by weight. The intake of the fatty acid menthol ester may vary depending, for example, upon the type of mammals, sex, growth, and weight. For example, the intake may be not less than 1 mg per day per adult (60 kg). The upper limit of the intake is not particularly limited but may be 20 g. Preferably, the lower limit of the intake is 0.1 g, and the upper limit of the intake is about 10 g.

The composition for improving lipid metabolism according to the present invention may be in any shape and may be, for example, liquid, solid, semi-solid, or gel. The composition for improving lipid metabolism according to the present invention comprises a fatty acid menthol ester as an active ingredient. The composition for improving lipid metabolism, however, may comprise any other optional ingredient. Any optional ingredient may be used so far as the ingredient can be safely ingested by mammals. Specifically, the optional ingredient may be the same as those described below in connection with the food and drink, health (supplementary) food, feed, or pharmaceutical composition which will be described later.

### Food and drink

In a preferred embodiment of the present invention, there is provided food and drink comprising the composition for improving lipid metabolism according to the present invention. The food and drink comprising the composition for improving lipid metabolism according to the present invention may be in any form and may be, for example, in the form of liquids, solids, semi-solids, gummy candies, or jellies. Specific examples of food and drink comprising the composition for improving lipid metabolism according to the present invention include: beverage foods, for example, liquid beverages, juice beverages, cold beverages, sport drinks, alcoholic beverages, teas, and nutritional supplemental beverages; bread, noodle, rice, confectionary (biscuit, cake, candy, chocolate, and Japanese confectionery), bean curd, and other processed foods; sake, distilled spirits, liqueur, alcoholic beverages for medicinal use, sweat sake, vinegar, soy sauce, bean paste and other fermented foods; fat-and-oil foods, for example, edible fats and oils; farm processed foods, for example, yogurt, ham, bacon, and sausage; fish-paste products, for example, fish sausage, deep-fried ball of fish paste, and pounded fish cake, and dried marine products or other fishery processed products.

In a preferred embodiment of the present invention, fat-and-oil products (fatty acids and glycerides) are proposed as the food and drink comprising the composition for improving lipid metabolism according to the present invention. The fat-and-oil food according to the present invention may be utilized not only as the above edible fats and oils but also as edible fat-and-oil compositions (for example, vegetable fats and oils, animal fats and oils, and processed fats and oils), and edible emulsified fat and oil compositions (for example, butter, lard, mayonnaise, cream, shortening, and margarine), and cooking additives (for examples, packed lunch and daily dishes).

In the food and drink according to the present invention, the content of the fatty acid menthol ester is approximately more than 0% by weight and not more than 100% by weight, based on the total weight of the food and drink. Preferably, the lower limit of the content is about 5% by weight, and the upper limit of the content is about 90% by weight. More preferably, the lower limit of the content is about 50% by weight, and the upper limit of the content is about 80% by weight.

### Health (supplementary) food

In a preferred embodiment of the present invention, there is provided a health (supplementary) food comprising the composition for improving lipid metabolism according to the present invention. The health (supplementary) food according to the present invention may be a cooking ingredient comprising the composition for improving lipid metabolism according to the present invention. Specific examples of cooking ingredients may be the same as those described above in connection with the food and drink. The health (supplementary) food according to the present invention may be a mixture of the composition for improving lipid metabolism according to the present invention with materials, which has food safety, for example, vehicles, extenders, binders, thickeners, emulsifiers, perfumes, acidulants, antiseptics, antioxidants, thickeners, colorants, food additives, and flavoring materials. In the case of the health supplementary food, for example, royal jelly, vitamins, amino acids, protein, chitosan, and lecithin may be incorporated as auxiliary substances, and carbohydrate solutions may be further added.

The health (supplementary) food according to the present invention may be formulated by a conventional method, for example, into tablets, soft capsules, hard capsules, granules, solid formulations, powder preparations, powders, pills, solvents, chewable preparations, drinkable preparations, dressings, and confectioneries.

In another embodiment of the present invention, there is provided a health (supplementary) food for improving lipid metabolism, comprising a fatty acid menthol ester as an active ingredient. The health (supplementary) food according to the present invention is provided in such a form that involves an indication about an improvement in lipid metabolism, for example, in packaging containers or packaging papers.

In the health (supplementary) food according to the present invention, the content of the fatty acid menthol ester is approximately more than 0% by weight and not more than 100% by weight based on the total weight of the health (supplementary) food. Preferably, the lower limit of the content of the fatty acid menthol ester is about 5% by weight, and the upper limit of the fatty acid menthol ester is about 90% by weight. More preferably, the lower limit of the fatty acid menthol ester is about 50% by weight, and the upper limit of the fatty acid menthol ester is about 80% by weight.

### Feed

In a preferred embodiment of the present invention, there is provided a feed comprising the composition for improving lipid metabolism according to the present invention. The feed according to the present invention may be a mixture of the composition for improving lipid metabolism according to the present invention with materials, which have feed safety, for example, vehicles, extenders, binders, thickeners, emulsifiers, perfumes, enzymes, microbial preparations, antioxidants, thickeners, colorants, feed additives, and flavoring agents. The feed according to the present invention may be formulated by a conventional method, for example, into solids, semi-solids, and liquids. The feed according to the present invention may be used for animals, particularly for domestic animals and pet animals.

In another embodiment of the present invention, there is provided a feed for improving lipid metabolism, comprising a fatty acid menthol ester as an active ingredient. The feed according to the present invention is provided in such a form that involves an indication about an improvement in lipid metabolism, for example, in packaging containers or packaging papers.

In the feed according to the present invention, the content of the fatty acid menthol ester is approximately more than 0% by weight and not more than 100% by weight, based on the total weight of the feed. Preferably, the lower limit of the content is about 1% by weight, and the upper limit of the content is about 50% by weight. More preferably, the lower limit of the content is about 5% by weight, and the upper limit of the content is about 20% by weight.

### Pharmaceutical composition

In a further embodiment of the present invention, there is provided a pharmaceutical composition for improving lipid metabolism, comprising a fatty acid menthol ester as an active ingredient.
The details of the fatty acid menthol ester may be the same as those described above in connection with the composition for improving lipid metabolism. The pharmaceutical composition according to the present invention is preferably used for preventing, improving or treating one or at least two symptoms selected from the group consisting of atherosclerosis, obesity, hypercholesterolemia, hypertriacylglycerolemia, hyperlipidemia, fatty liver, diabetes, and hypertension.

From the viewpoint of more reliably attaining the effect of the pharmaceutical composition for improving lipid metabolism, preferably the pharmaceutical composition for improving lipid metabolism is produced by mixing the fatty acid menthol ester as an active ingredient with a properly selected pharmaceutically acceptable carrier or additive, for example, vehicles, extenders, binders, wetting agents, disintegrators, surfactants, lubricants, dispersants, buffers, preservatives, solubilizers, flavoring agents, soothing agents, corrigents, and stabilizers.

Specific examples of vehicles include lactose, saccharose, glucose, sodium hydrogencarbonate, sucrose, mannitol, starch, crystalline cellulose, calcium sulfate, calcium phosphate, ethyl cellulose, and methacrylate copolymer. Specific examples of binders include gum arabic, polyvinyl pyrrolidone, hydroxypropylcellulose, hydroxypropylmethylcellulose, carboxymethylcellulose, gelatin, glucose, saccharose, tragacanth, and sodium alginate. Specific examples of disintegrators include starch, crystalline cellulose, carboxymethylcellulose, sodium carboxymethyl starch, sodium carboxymethylcellulose, sodium croscarmellose, crospovidone, and low substituted hydroxypropylcellulose. Specific examples of surfactants include polyoxyethylene alkyl ethers, polyoxyethylene sorbitan fatty acid esters, polyoxyethylene glyceryl monofatty acid esters, polyoxyethylene propylene glycol monofatty acid esters, polyoxyethylene sorbitol fatty acid esters, polyoxyethylene derivatives of naturally occurring fats and oils and waxes, polyethylene glycol fatty acid esters, sorbitan fatty acid esters, sucrose fatty acid esters, polyoxyethylene-polyoxypropylene copolymer and block copolymer surfactants, alkyl sulfate ester salts, phospholipid, bile acid salts, fatty acids, monohydric alcohol fatty acid esters, ethylene glycol fatty acid esters, and polyhydric alcohol fatty acid esters. Specific examples of preservatives include p-oxybenzoic esters, chlorobutanol, and banzyl alcohol. Specific examples of colorants include those commonly used in the field of pharmaceuticals and foods. Examples of corrigents include aminoethyl sulfonic acids, sodium alginate, and ethanol.

The pharmaceutical composition for improving lipid metabolism according to the present invention can be administered to animals including human through any of oral and parenteral administration (for example, rectal or percutaneous) routes. Accordingly, the pharmaceutical composition for improving lipid metabolism according to the present invention is preferably provided as a proper dosage depending upon the administration route. For example, the pharmaceutical composition for improving lipid metabolism according to the present invention can be formulated into various preparations, for example, injections, capsules, tablets, soft capsules, hard capsules, chewable preparations, solid formulations, granules, powder preparations, solvents, pills, fine subtilaes, drinkable preparations, ointments, creams, troches or other oral preparations, and preparations for rectal administration.

In the pharmaceutical composition for improving lipid metabolism according to the present invention, the content of the fatty acid menthol ester is approximately more than 0% by weight and not more than 100% by weight based on the total weight of the pharmaceutical composition. Preferably, the lower limit of the content is about 5% by weight, and the upper limit of the content is about 90% by weight. More preferably, the lower limit of the content is about 50% by weight, and the upper limit of the content is about 80% by weight.

The dose of the pharmaceutical composition for improving lipid metabolism according to the present invention may vary depending, for example, upon therapeutic purposes and therapeutic objectives and thus cannot be determined unconditionally. From the viewpoint of therapeutic effectiveness, for example, for an adult having a weight of 60 kg, the dose per day is not less than about 10 mg and not more than about 5 g in terms of the dose of the fatty acid menthol ester. Preferably, the lower limit of the dose is about 20 mg, and the upper limit of the dose is about 3 g.

### EXAMPLES

The present invention is further illustrated by the following Examples that are not intended as a limitation of the invention.

### Example 1

A test medium containing 150 µM of linolic acid menthol ester (1% BSA-DMEM) was provided. In this test medium, human liver-derived HepG2 cells were cultured for 24 hr, and the cells and the medium were collected.

### Comparative Example 1

The procedure of Example 1. was repeated to collect the cells and medium, except that linolic acid was used instead of the linolic acid menthol ester.

### Comparative Example 2

The procedure of Example 1 was repeated to collect the cells and medium, except that conjugated linolic acid was used instead of the linolic acid menthol ester.

### Evaluation tests

The cells and media obtained in the Examples and Comparative Examples were evaluated by the following tests.

### Evaluation 1: Evaluation test on toxicity of test medium

The toxicity of the test medium was determined by examining the mass of cell proteins measured by the MTT and BCA methods. As a result, it was found that there was no cytotoxicity at a concentration of 150 µM.

### Evaluation 2: Test on secretion (inhibition/enhancement) of apolipoproteins A and B

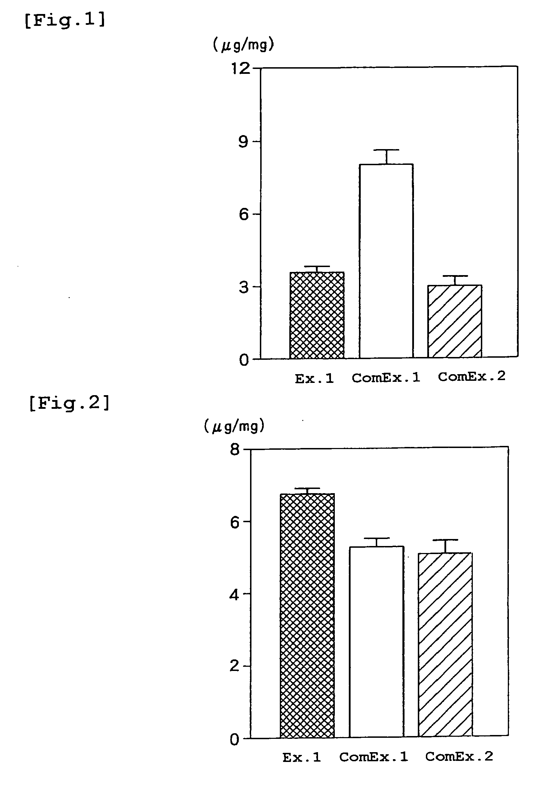

The amount of apolipoprotein A (A1) and apolipoprotein B (B100) secreted from the human liver-derived HepG2 cells into the test medium was measured by the ELISA method. The results are shown in Figs. 1 and 2. As shown in Figs. 1 and 2, the linolic acid menthol ester was found to inhibit the secretion of apolipoprotein B (B100) (Fig. 1) and was further found to significantly enhance the secretion of apolipoprotein A (A1) (Fig. 2).

### Evaluation results

The above results show that the fatty acid (linolic acid) menthol ester has the function of inhibiting the secretion of apolipoprotein B (B100), enhancing the secretion of apolipoprotein A (A1), and improving lipid metabolism.

## Claims

1. A composition for improving lipid metabolism, comprising a fatty acid menthol ester as an active ingredient.

2. The composition for improving lipid metabolism according to claim 1, wherein the fatty acid menthol ester is represented by general formula (I): 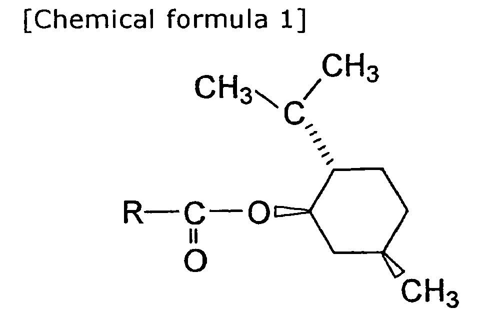 wherein
R represents a saturated fatty acid residue, a monoenoic acid residue, a polyenoic acid residue, or a hydroxy acid residue.

3. The composition for improving lipid metabolism according to claim 1, wherein the fatty acid constituting the fatty acid menthol ester is one or at least two fatty acids selected from the group consisting of fatty acids having not less than 8 and not more than 24 carbon atoms.

4. The composition for improving lipid metabolism according to claim 3, wherein the fatty acid is one or at least two fatty acids selected from the group consisting of octanoic acid, decanoic acid, dodecanoic acid, tetradecanoic acid, palmitic acid, stearic acid, arachidic acid, behenic acid, ligceric acid, palmitoleic acid, oleic acid, elaidic acid, eicosapentaenoic acid, docosahexaenoic acid, linoleic acid, conjugated linoleic acid, γ-linolenic acid, α-linolenic acid, conjugated linolenic acid, arachidonic acid, 2-hydroxylignoceric acid, and hydroxynervonic acid.

5. The composition for improving lipid metabolism according to any one of claims 1 to 4, wherein the fatty acid menthol ester inhibits the secretion of apolipoprotein B and/or promotes the secretion of apolipoprotein A.

6. The composition for improving lipid metabolism according to claim 5, wherein the secretion of the apolipoprotein B is inhibited in the liver and/or the secretion of the apolipoprotein A is promoted in the liver and/or intestinal tract.

7. The composition for improving lipid metabolism according to claim 5, wherein the concentration of triacyl glycerol, cholesterol and/or cholesterol esters in blood, lymphs and/or body tissues is lowered by inhibiting the secretion of the apolipoprotein B, and/or
lecithin:cholesterolacyl transferase is activated by promoting the secretion of the apolipoprotein A to promote the degradation of cholesterol in blood, lymphs and/or body tissues.

8. The composition for improving lipid metabolism according to claim 5, wherein the concentration of triacyl glycerol, cholesterol and/or cholesterol esters in blood, lymphs and/or body tissues is lowered.

9. The composition for improving lipid metabolism according to claim 5, which lowers the concentration of low-density lipoprotein in blood, lymphs and/or body tissues, and/or
increases the concentration of high-density lipoprotein in blood, lymphs and/or body tissues.

10. The composition for improving lipid metabolism according to any one of claims 1 to 4, for use in the prevention or treatment of one or at least two symptoms selected from the group consisting of atherosclerosis, obesity, hypercholesterolemia, hypertriacylglycerolemia, hyperlipemia, fatty liver, diabetes mellitus, and hypertension.

11. Food and drink comprising a composition for improving lipid metabolism according to any one of claims 1 to 4.

12. A health food comprising a composition for improving lipid metabolism according to any one of claims 1 to 4.

13. A feed comprising a composition for improving lipid metabolism according to any one of claims 1 to 4.

14. A pharmaceutical composition for improving lipid metabolism, comprising a fatty acid menthol ester as an active ingredient.

15. The pharmaceutical composition according to claim 14, wherein the fatty acid menthol ester is as defined in any one of claims 2 to 4.

16. The pharmaceutical composition according to claim 14, for use in the prevention or treatment of one or at least two symptoms selected from the group consisting of atherosclerosis, obesity, hypercholesterolemia, hypertriacylglycerolemia, hyperlipemia, fatty liver, diabetes mellitus, and hypertension.
